Europäisches Patentamt

European Patent Office

Office européen des brevets

(11) Publication number: **0 200 541**
**B1**

# EUROPEAN PATENT SPECIFICATION

(45) Date of publication of patent specification: **03.10.90**

(21) Application number: **86303272.8**

(22) Date of filing: **30.04.86**

(51) Int. Cl.⁵: **G 01 N 33/573**, G 01 N 33/52
// C12Q1/50

(54) Immunochemical method and analytical composition and element for determination of creatine kinase-MB.

(30) Priority: **01.05.85 US 729333**

(43) Date of publication of application:
**05.11.86 Bulletin 86/45**

(45) Publication of the grant of the patent:
**03.10.90 Bulletin 90/40**

(84) Designated Contracting States:
**CH DE FR GB LI**

(56) References cited:
EP-A-0 015 437    WO-A-82/02213
EP-A-0 022 432    US-A-4 235 961
EP-A-0 071 087

CLINICAL CHEMISTRY, vol. 28, no. 4, April 1982,
pages 609-614, Washington, US; A. LUNDIN et
al.: "Optimized bioluminescence assay of
creatine kinase and creatine kinase B-subunit
activity"

(73) Proprietor: **EASTMAN KODAK COMPANY (a
New Jersey corporation)
343 State Street
Rochester New York 14650 (US)**

(72) Inventor: **Findlay, John Bruce
Kodak Park
Rochester New York 14650 (US)**
Inventor: **Wu, Annie Liu
Kodak Park
Rochester New York 14650 (US)**

(74) Representative: **Nunney, Ronald Frederick
Adolphe et al
Kodak Limited Patent Department Headstone
Drive
Harrow Middlesex HA1 4TY (GB)**

(56) References cited:
CLINICAL CHEMISTRY, vol. 28, no. 7, July 1982,
pages 1439-1447, Washington, US; H. LANG et
al.: "Creatine kinase, an enzyme of many forms"

CLINICAL CHEMISTRY, vol. 22, no. 11, 1976,
pages 1806-1811, Washington, US; G. SZASZ et
al.: "Creative kinase in serum: 2. Interference of
adenylate kinase with the assay"

Courier Press, Leamington Spa, England.

**Description**

The present invention relates to clinical chemistry. In particular, it relates to a method for the determination of creatine kinase-MB in aqueous liquids, e.g. biological fluids. This invention also relates to an analytical composition and element which is useful in such method.

The determination of the activity of creatine kinase (abbreviated herein to CK, but also known as creatine phosphokinase, CPK, or ATP:creatine phosphotransferase E.C.2.7.3.2.) in human serum is considered one of the most sensitive laboratory methods for diagnosing diseases of skeletal muscles, such as diseases of the myocardium. CK determination is useful, for example, for diagnosis of progressive muscular dystrophy, dermatomyositis, and especially myocardial infarctions.

CK occurs in human body fluids and tissue in the form of three isoenzymes: CK-MM, for example in muscles, CK-BB, for example in the brain, and CK-MB, for example in the myocardium. The CK activity occurring in healthy human blood serum is normally due to the CK-MM isoenzyme, because CK-BB does not generally pass into the blood stream. In a healthy individual, CK-MB is generally restricted to certain organs, for example the myocardium. However, when the myocardium is damaged, as in the case of a cardiac infarction, CK-MB is released into the blood serum and can be detected therein.

A potential difficulty encountered in methods for determining CK-MB in biological fluids is interference from the other two isoenzymes. For practical purposes, the amount of CK-BB in the fluid is considered negligible in most determinations. In methods for determining CK-MB, it is known to precipitate or inhibit the M subunit with specific antibodies to eliminate the interference of CK-MM and then to measure the remaining hybrid isoenzyme CK-MB.

Standard assays for total creatine kinase (i.e. assay for all isoenzymes) generally use either the forward or reverse reaction illustrated by the following equation:

$$\text{1.} \quad \text{creatine} + \text{adenosine-5'-triphosphate (ATP)} \overset{\text{CK}}{\rightleftharpoons} \text{creatine phosphate} + \text{adenosine-5'-diphosphate (ADP)}.$$

Both the forward and reverse reactions have been used in analytical procedures, but use of the reverse reaction is preferred because it is about six times faster than the forward reaction. The presence of ATP can then be determined by a number of colorimetric or potentiometric methods.

A recognized interferent in the determination of total CK using equation (I) is adenylate kinase (AK) which is always present in normal human sera, but especially in hemolyzed samples of sera. AK is also present as a number of isoenzymes which are identified as to their source, e.g. erythrocyte, muscle and liver. AK catalyzes the formation of ATP according to the following equation taken in the forward direction:

$$\text{2.} \quad \text{ADP} \overset{\text{AK}}{\rightleftharpoons} \text{AMP} + \text{ATP}$$

wherein AMP is adenosine-5'-monophosphate. Thus, the presence of AK causes a detectable apparent increase in the amount of ATP in a serum sample, and consequently would give an erroneous apparent increase in CK activity according to the reverse of equation (I).

In one standard creatine kinase or creatine kinase-MB assay, as described in EP—A—0 071 087, most adenylate kinase activity is suppressed by means of an AMP:ADP ratio of at least 10:1 in combination with 5 to 8 millimolar fluoride ion. Another known AK inhibitor, $P^1,P^5$-di(adenosine-5')pentaphosphate (DAPP) is known to provide insufficient AK inhibition. Solution CK-MB assays require blanking, whereby AK activity is separately measured and subtracted from the test assay results, because complete AK inhibition is not obtained with the combination of AMP and DAPP. A blanking procedure is highly disadvantageous, however, for use in highly automated clinical chemistry analyzers and procedures because it complicates equipment and procedures and increases the likelihood of error.

The use of fluoride ion as an AK inhibitor has its disadvantages. Fluoride tends to precipitate as a salt in the presence of magnesium ion, a common enzyme cofactor used in CK assays. Its inhibition of AK is slow, requiring up to ten minutes which is an undesirably long time. Further, fluoride tends to inhibit CK activity as well. As a result, a number of researchers have avoided fluoride ion and turned to DAPP and AMP to inhibit AK activity.

Szasz et al, in *Clin. Chem., 22* (11), pp. 1806—1811 (1976), describe investigations of AK inhibition in total CK assays. This reference teaches that erythrocyte and muscle AK are 97% inhibited by means of 0.01 millimolar DAPP. However, only 80% of the liver isoenzyme is inhibited. An AMP:ADP molar ratio of 10:1 inhibits 95% of total AK, but it also reduces the activity of the analyte, total CK, by 15—20%. With 0.5—1 millimolar DAPP alone, up to 97% of the activity of liver AK is inhibited, but only at the expense of a 5% reduction in CK activity. Further, the researchers discourage use of DAPP because of its high cost at the higher concentrations. Hence, these researchers concluded that, for total CK determinations, a suitable compromise for AK inhibition would be 0.01 millimolar DAPP and a 5:2 molar ratio of AMP to ADP.

This research became the basis for the total CK assay standard adopted in recent years. This standard assay reduces the interference by erythrocyte and muscle AK by 97%, but liver AK is reduced only by 90—95%. At the same time, 5% of CK activity is sacrificed. In order to overcome this serious defect, serum blanks are generally run to determine the residual AK activity. However, running serum blanks is tedious, complicates the assay and increases the likelihood of error.

The levels of inhibitors (e.g. 0.01 DAPP and 5:2 molar ratio AMP:ADP) taught in the noted art are often sufficient to reduce AK interference to sufficient levels for total CK determinations. However, in an assay for CK-MB in which other creatine kinase isoenzymes are rendered inactive, the amount of CK available for reaction is considerably smaller. Hence, determination (i.e. quantification) of the remaining CK is much more sensitive to interference by adenylate kinase or any reagent which would significantly affect the CK measurement.

According to the teaching of the art, to reduce AK interference 97%, a worker in the art would use 0.5—1 millimolar DAPP as an AK inhibitor. However, this level of DAPP also reduces the activity of CK-MB by at least 5% which is an unacceptable amount. Combining a lower amount of DAPP with a molar ratio of AMP:ADP of 10:1 reduces CK activity even further. It appears that the art would suggest avoiding high DAPP levels and high AMP-ADP ratios. Yet, in the determination of CK-MB, it would be highly desirable to eliminate at least 99% of all AK interference, especially liver AK, without a decrease in CK activity in order to render the assay highly accurate and sensitive to the CK-MB isoenzyme.

A bioluminescent assay for creatine kinase-MB is described by Lundin et al, *Clin. Chem,* 28(4), pp. 609—614 (1982). In this assay, DAPP is used at 0.2 µmolar to inhibit adenylate kinase and an AMP:ADP ratio of 2:1. While this combination of reagents is considered to provide an optimized bioluminescent assay, it fails to suggest means to reduce the activity of all AK isoenzymes in an accurate and sensitive CK-MB assay, whether bioluminescent or spectrophotometric.

The problems noted above have been overcome with an analytical composition for the determination of creatine kinase-MB while substantially all activity of isoenzymes of adenylate kinase is inhibited, the analytical composition comprising creatine phosphate and an indicator composition which is capable of providing a detectable change in response to reaction of creatine phosphate or its reaction product,

the composition being characterized by comprising, in the absence of fluorine ion, adenosine-5'-diphosphate in an amount of from 1 to 3 millimolar, and

a combination of adenylate kinase inhibitors consisting essentially of:

(a) $P^1,P^5$-di(adenosine-5')polyphosphate present in an amount of at least 0.3 millimolar, and

(b) adenosine-5'-monophosphate present in a molar ratio to adenosine-5'-diphosphate of at least 10:1.

A dry analytical element for the determination of creatine kinase-MB comprises an absorbent carrier material and contains the analytical composition described above.

This invention further provides a method for the determination of creatine kinase-MB in an aqueous liquid comprising the steps of:

(A) in the presence of at least one antibody for creatine kinase-MM, contacting a sample of a liquid suspected of containing creatine kinase-MB with the analytical composition described above, and

(B) determining the detectable change resulting from the presence of creatine kinase-MB.

The present invention provides an assay and element for creatine kinase-MB in aqueous liquids which overcome the problems noted above. In particular, the assay of this invention is not susceptible to interference by adenylate kinase to any significant degree, thereby rendering it highly accurate and sensitive to CK-MB. In particular, at least 99% of liver AK activity is inhibited with this invention. Further, it is simple to use. The assay of this invention can be carried out with undiluted samples if desired and sample blanking is unnecessary. These advantages are achieved by use of critical amounts of the AK inhibitors $P^1,P^5$-di(adenosine-5')polyphosphate, e.g. DAPP, and AMP in the assay, in the absence of fluoride ion.

The present invention relates to the determination (qualitative or quantitative measurement) of creatine kinase-MB in aqueous liquids. In particular, the invention can be used to assay biological fluids of either animals or humans, but preferably of humans. Such fluids include, but are not limited to, whole blood, plasma, sera, lymph, bile, urine, spinal fluid, sputum and perspiration as well as stool secretions. It is also possible to assay fluid preparations of human or animal tissue such as skeletal muscle, heart, kidney, lungs, brains, bone marrow and skin. The preferred biological fluid for practice of the invention is human blood serum. Advantageously, the test sample can be diluted or undiluted.

In particular, the present invention relates to an immunochemical method for selectively determining creatine kinase-MB in a biological fluid which also possibly contains CK-MM and CK-BB. Generally, the method comprises contacting the liquid to be assayed with the analytical composition of this invention, the details of which are provided below. Prior to or simultaneously with that contact, the liquid sample is contacted with one or more antibodies which are capable of either preferentially reacting with or preferentially inhibiting the enzymatic activity of the M subunits in the CK-MM and CK-MB isoenzymes present in the sample. The B subunits of the CK-MB isoenzyme are ideally uneffected by the presence of the antibodies, and therefore, are free to react in any of a number of reaction schemes to produce a detectable change. The amount of CK-BB is generally considered negligible in such assays. The detectable change produced is then directly correlated to the amount of CK-MB in the fluid sample.

The detectable change can be either a potentiometric change or an optical density change as long as the change results from the reaction of creatine phosphate or its reaction product according to the reaction (1) in the forward direction:

$$\text{(1)} \qquad \text{Creatine phosphate} + \text{ADP} \xrightleftharpoons[]{\text{CK-MB}} \text{creatine} + \text{ATP}.$$

In its simplest form, the assay can measure either the disappearance of creatine phosphate, or the appearance of creatine as described, for example, in PCT Application 82/2213.

Generally, however, reaction (1) is coupled with one or more other enzymatic reactions which provide a potentiometric change or an optical density change as a result of further reaction of ATP or its reaction product. If a potentiometric change is generated, CK-MB is determined by measuring the rate of change in electrical output with an ion-sensing electrode. For example, U.S. Patent 4,042,462 describes a potentiometric means for measuring the uptake of oxygen as a result of the following sequence of enzymatic reactions (2)—(4) used in combination with reaction (1) above:

$$\text{(2)} \qquad \text{ATP} + \text{glucose} \xrightarrow{\text{hexokinase}} \text{glucose-6-phosphate} + \text{ADP}$$

$$\text{(3)} \qquad \text{glucose-6-phosphate} + \text{NADP}^+ \xrightarrow{\text{G-6-PDH}} \text{6-phosphogluconate} + \text{NADPH} + \text{H}^+$$

$$\text{(4)} \qquad \text{NADPH} + \text{H}^+ + 1/2\ \text{O}_2 \xrightarrow{\text{ETA}} \text{NADP} + \text{H}_2\text{O}$$

where NADP (NADPH) is nicotinamide adenine dinucleotide phosphate (same, reduced form), G-6-DPH is glucose-6-phosphodehydrogenase and ETA is an electron transfer agent.

Preferably, CK-MB is determined in the present invention by measuring an optical density change. This change can be colorimetric, fluorometric, or photometric, and is meant to include the rate of appearance of light, the rate of increase or decrease in optical density, and the rate of a shift in absorbance from one wavelength to another. For example, a photometric assay is described in U.S. Patent 4,235,961 wherein reaction (1) above is combined with the following reaction (5) to produce detectable emissions of light:

$$\text{(5)} \qquad \text{ATP} + \text{luciferin} + \text{O}_2 \xrightarrow{\text{luciferase}} \text{oxyluciferin} + \text{CO}_2 + \text{AMP} + \text{PPi} + \text{light}$$

wherein PPi is pyrophosphate.

More particularly, CK-MB is determined by colorimetric means whereby an optical density change is measured at a wavelength between 200 and 900 nm. At wavelengths below 400 nm, the measurements are generally in the ultraviolet regions of the electromagnetic spectrum, as when NADH (i.e. nicotinamide adenine dinucleotide, reduced from) or NADPH are measured according to the following coupled enzymatic reaction schemes.

Reactions (1), (2) and (3) noted above can be used together, as described in *J. Clin. Chem. Clin. Biochem.*, *15*, pp. 255—260 (1977) and U.S. Patent 4,220,714.

A similar sequence of coupled reactions beginning with reaction (1) and using NADH in reactions (6) and (7) below is described in U.S. Patent 4,352,881:

$$\text{(6)} \qquad \text{ATP} + \text{3-phosphoglycerate} \xrightarrow{\text{phosphoglycerate kinase}} \text{ADP} + \text{1,3-diphosphoglycerate}$$

$$\text{(7)} \qquad \text{1,3-diphosphoglycerate} + \text{NADH} \xrightarrow{\text{glyceraldehyde-3-phosphate dehydrogenase}}$$

$$\text{glyceraldehyde-3-phosphate} + \text{NAD}^+$$

Other colorimetric assays carry either reaction (3) or (7) further by reducing a colorless tetrazolium salt and forming a colored dye according to reaction (8):

$$\text{(8)} \qquad \text{NADH (or NADPH)} + \text{tetrazolium salt} \xrightarrow[\text{diaphorase}]{\text{ETA or}} \text{NAD}^+ \text{(or NADP}^+\text{)} + \text{formazan dye}$$
$$\text{(colorless)}$$

Such assays are described, for example, in U.S. Patents 4,012,286 and 4,247,633.

The reagents and enzymes needed for the assays described above are commercially available. In each case, the indicator composition comprises the nonenzymatic reagents needed for the assay.

4

In a preferred embodiment of the present invention, creatine kinase-MB (CK-MB) activity is determined by the following sequence of reactions:

(1)      Creatine phosphate+ADP $\xrightarrow{\text{CK-MB}}$ creatine+ATP

(9)      ATP+glycerol $\xrightarrow{\text{glycerol kinase}}$ L-α-glycerolphosphate+ADP

(10)     L-α-glycerophosphate+electron acceptor $\xrightarrow{\text{α-glycerophosphate oxidase}}$

colorimetrically detectable species.

In these combined reactions, the rate of formation of the colorimetrically detectable species is directly proportional to the rate of creatine kinase-MB activity in the liquid sample. The details of this sequence of reactions are provided in EP Application 116,307.

The first reaction in the above-described sequence is the reaction of creatine phosphate and ADP to form creatine and ATP in the presence of creatine kinase-MB in the aqueous liquid sample. As is well known in the art, this reaction usually proceeds in the presence of an enzyme cofactor, such as a divalent metal ion. Exemplary cofactors are described below. Creatine phosphate is a biological compound commercially available from any of a number of sources. Creatine phosphate is used in the practice of invention in an amount in excess of that needed stoichiometrically to make the reaction proceed.

ADP is the hydrolyzed form of the nucleotide ATP. The amount of ADP used in the assay is discussed in more detail below.

As shown in reaction (9) above, glycerol kinase catalyzes the phosphorylation of glycerol to L-α-glycerophosphate in the presence of ATP. Generally, any glycerol kinase is useful in the successful practice of the present invention although those obtained from *E. coli* and *Candida mycoderma* are preferred. Glycerol kinase enzymes from other sources are well known in the art.

The glycerol useful in the practice of this invention can also be readily obtained commercially or prepared using techniques well known in the art. Glycerol can be provided either in free form or as a fatty acid ester of glycerol (e.g. triglycerides).

The next step in the reaction sequence involves the oxidation of L-α-glycerophosphate and, generally, an electron acceptor to produce a colorimetrically detectable species. This species is quantitatively related to the creatine kinase-MB contained in the liquid sample.

L-α-glycerophosphate oxidase is a microbial enzyme which can be derived from a variety of sources. A detailed description of this enzyme and exemplary sources are provided in U.S. Patent 4,241,178.

The oxidation of L-α-glycerophosphate occurs in the presence of an electron acceptor. Any electron acceptor which will permit oxidation of the phosphate by the oxidase with the concomitant production of a colorimetrically detectable species is suitable for use in this invention.

In one embodiment, the electron acceptor can be an indicator composition containing a chromogen (which is defined in more detail below). Such chromogen can be reduced to provide an optical density change (as defined above). The rates of any of these changes can then be monitored to measure creatine kinase-MB activity. Certain indophenols, potassium ferricyanide and certain tetrazolium salts are useful as electron acceptors in the practice of this embodiment. For example, 26-dichlorophenolindophenol alone or in combination with phenazine methosulfate or phenazine ethosulfate (i.e. an electron transfer agent), and 2-(p-indophenyl)-3-(p-nitrophenyl)-5-phenyl-2H-tetrazolium chloride either alone or in combination with phenazine methosulfate is especially useful.

In an alternative and preferred embodiment, the electron acceptor oxidizes the phosphate to produce an intermediate species which then reacts with an indicator composition to produce a colorimetrically detectable species according to the following reactions:

(10a)        L-α-glycerophosphate+electron acceptor $\xrightarrow{\text{α-glycerophosphate oxidase}}$

dihydroxyacetone phosphate+intermediate species (e.g. $H_2O_2$)

Intermediate species+colorimetric indicator composition $\longrightarrow$ colorimetrically detectable species.
(10b)

Quantification of creatine kinase in the practice of this preferred embodiment is achieved using oxygen as the electron acceptor and a colorimetric indicator composition which comprises: (1) a substance having peroxidative activity, and (2) a chromogen. In such a case, reaction (10a) produces dihydroxyacetone phosphate and hydrogen peroxide.

Colorimetric indicator compositions which react with hydrogen peroxide in reaction (10b) are well known in the art. Generally, such compositions comprise a substance which has peroxidative activity. Preferably, this substance is peroxidase.

A peroxidase is an enzyme which will catalyze a reaction wherein hydrogen peroxide oxidizes another substance. The peroxidases are generally conjugated proteins containing iron porphyrin. Peroxidase occurs in horseradish, potatoes, figtree sap and turnips (plant peroxidase), in milk (lacto peroxidase), and in white blood corpuscles (verdo peroxidase). It also occurs in microorganisms and can be produced by fermentation. Certain synthetic peroxidases, such as those disclosed by Theorell and Maehly in *Acta Chem. Scand.*, Vol. 4, pages 422—434 (1950), are also useful. A preferred peroxidase is than obtained from horseradish.

Also, useful but to a lesser extent are such substances as hemin, methemoglobin, oxyhemoglobin, hemoglobin, hemochromogen, alkaline hematin, hemin derivatives, iron sulfocyanate, iron tannate, ferrous ferrocyanide, chromic salts (such as potassium chromic sulfate) absorbed in e.g. silica gel.

The colorimetric indicator composition also comprises a chromogen which is a colored or colorless substance which directly or indirectly provides a quantifiable colorimetric change the rate of which change can be quantiatively measured. The chromogen can be a dye, dye former or dye precursor. The color provided by the reaction of the chromogen is in the visible region of the electromagnetic spectrum (i.e. between 400 and 900 nm).

Chromogens which provide color formation in the presence of hydrogen peroxide and peroxidase useful in the present invention include (with a color coupler where necessary): monoamines, diamines, phenols, polyphenols, aromatic acids, leuco dyes and triarylmethane dyes.

Other chromogens which contain a material oxidizable in the presence of peroxidase and which can provide a colorimetrically detectable species include certain dye-providing compositions. In one aspect, such chromogens can include a compound that, when oxidized by peroxidase, can couple with itself or with its reduced form to provide a dye. Such autocoupling compounds include a variety of hydroxylated compounds which are well known in the art.

In another aspect, the detectable species can be provided by chromogens which include a peroxidase-oxidizable compound capable of undergoing oxidative condensation with couplers such as those containing phenolic groups or activated methylene groups, together with such a coupler. Representative of such oxidizable compounds are benzidene and its homologs, p-phenylenediamines, p-aminophenols and 4-aminoantipyrine. A wide range of such couplers, including a number of autocoupling compounds, is described in the art. A wide range of color couplers, including autocoupling compounds, are known in the art, including N,N-disubstituted toluidines, dihydroindoles and tetrahydroquinolines.

In still another and preferred aspect, the colorimetrically detectable species can be provided by peroxidase-induced oxidation of a leuco dye to provide the corresponding dyestuff form.

A variety of leuco dyes are useful as chromogens in the practice of this invention including those described in U.S. Patents 4,241,178. Leuco dyes most preferred for use in this invention are the triarylimidazoles of U.S. Patent 4,089,747.

Particularly useful leuco dyes include 2-(3,5-dimethoxy-4-hydroxyphenyl)-4,5-bis(4-dimethylaminophenyl)imidazole, 2-(4-hydroxy-3-methoxyphenyl)-4,5-bis(p-dimethylaminophenyl)-1H-imidazole and 2-(3-ethoxy-4-hydroxyphenyl-4,5-bis(p-dimethylaminophenyl)-1H-imidazole.

The amounts of the components of the indicator compositions useful in the practice of this invention, including the preferred colorimetric indicator compositions, are dependent to a large extent upon the concentration of creatine kinase-MB in the sample, the sophistication of the detection apparatus, and the detectable change produced. The amounts are readily determinable by a worker skilled in clinical chemistry.

The assay of this invention uses a critical combination of the adenylate kinase (AK) inhibitors:

a) $P^1,P^5$-di(adenosine-5')polyphosphate, and

b) adenosine-5'-monophosphate (AMP), in proportions found to be effective to substantially reduce the activity of all (i.e. at least 99%) isoenzymes of endogenous adenylate kinase (AK) so that AK does not affect the determination of CK-MB. No other AK inhibitors (e.g. fluoride) are present in this combination. Fluoride is excluded from the combination because it also inhibits CK activity and tends to precipitate in the presence of magnesium ion, a common cofactor used in CK assays. Although the amounts to achieve the critical reduction in AK activity can vary, generally, the polyphosphate is present in an amount of at least 0.3 millimolar, and preferably at least 0.5 millimolar. No upper limit is critical although for practical purposes, no more than 1 millimolar of the polyphosphate is used.

Generally, AMP and ADP are present in a molar ratio of at least 10:1 with ADP present in an amount of from 1 to 3 millimolar. Preferably, the molar ratio is at least 12:1, and ADP is present in an amount of from 1 to 2 millimolar. More preferably, the AMP to ADP molar ratio is at least 14:1 and ADP is present in an amount of 1.5 millimolar.

The polyphosphate, which can be $P^1,P^5$-di(adenosine-5')tetra-, penta- or hexaphosphate, and AMP and ADP are readily available commercially. $P^1,P^5$-di(adenosine-5')pentaphosphate is particularly useful in the practice of this invention.

The analytical composition of this invention can also include other reagents or addenda generally used in total CK or CK-MB determinations, including CK activators, metal ion cofactors (e.g. magnesium, calcium and iron ions), solvents, buffers and surfactants. It is particularly desirable to include one or more CK activators which promote full creatine kinase activity. Such activators include mercapto-containing compounds (also known as thiol-containing or sulfhydryl compounds), such as thioglucose, dithiothreitol, dithioerythritol, mercaptoethanol, glutathione, N-acetylcysteine, cysteine, thioglycerol and thioglycolic acid, in amounts known to one skilled in clinical chemistry.

The element also advantageously includes ethylenebis(oxyethylenenitrilo)tetraacetic acid in a suitable amount to improve CK sensitivity by suppressing the inhibitory effect of calcium ions.

Antibodies useful in the practice of this invention which are specific to M subunits can be generated from antisera using known procedures. The antibodies can be isolated from the antisera before use, or unpurified antisera can be used. Antisera is generally obtained from suitably innoculated monkeys, pigs, horses, goats, rabbits, rats, mice, chickens, cattle, and other animals known to be useful for this purpose. A preferred source of antibodies are suitably inoculated goats. The antibodies are generally used in an immobilized form on a suitable carrier. In the assay of this invention, one or more antibodies can be immobilized within the element itself, if desired, without any additional carrier material or added prior to or simultaneously with the test sample to the element during the assay. Further details of useful antibodies and carrier materials are provided, for example, in U.S. Patents 4,237,044 and 4,260,678.

The CK-MB assay of this invention can be carried out either in solution or using a dry analytical element. In solution assay, generally the analytical composition and liquid test sample are mixed, in the presence of one or more antibodies for CK-MM, in a suitable container (e.g. test tube, petri dish, beaker, cuvette). An activator for CK can also be added to the reaction mixture to obtain optimum activity. The resulting reaction mixture can be incubated for a period of time at a suitable temperature if desired. The reaction mixture is then evaluated by measuring the resulting detectable change, if any, using standard detection equipment and procedures. The amount of change observed is then correlated to the amount of CK-MB in the liquid sample using standard procedures. The reaction mixture is generally buffered to a pH of 8 or less. Any suitable buffer or mixture thereof can be used in the practice of this invention.

The analytical composition, antibodies and any articles needed for the practice of this invention can be provided as part of a diagnostic kit for either dry or solution assays. For solution assays, the kit components can be supplied, individually or in combination in a container means, as lyophilized reagents in individual packets having predetermined amounts. Alternatively, they can be provided in bottled or otherwise packaged solutions sufficient in size for one or more assays. Other reagents or nonreactive addenda can also be supplied in the kit along with suitable assay utensils or containers for performing the assay, if desired. A dry analytical element (described below) can also be included as part of a diagnostic kit.

Alternatively, the reagents needed for the assay can be obtained from separate sources and brought together to provide an analytical composition.

The method of this invention can also be practiced with a dry analytical element. The simplest element can be composed of an absorbent carrier material, e.g. a thin sheet of a self-supporting absorbent or bibulous material, such as filter paper or strips, which contains the analytical composition of this invention. The element can be divided into two or more discrete zones with different reagents incorporated into individual zones of the carrier material. Such elements are known in the art as test strips, diagnostic elements, dip sticks and diagnostic agents. The element also preferably contains the antibodies for the M subunit described above.

The analytical composition can be incorporated into the absorbent carrier material by imbibition, impregnation, coating or another suitable technique. Generally, the composition is incorporated into a coating composition, whereas the CK-MM antibodies are incorporated by imbibition or wash coating into an already coated zone.

Useful absorbent carrier materials are insoluble and maintain their structural integrity when exposed to water or biological fluids such as whole blood or serum. Useful elements can be prepared from paper, porous particulate structures, porous polymeric films, cellulose, wood, glass fiber, woven and nonwoven fabrics (synthetic and nonsynthetic). Useful materials and procedures for making such elements are well known in the art as exemplified in U.S. Patents 3,092,465, 3,802,842, 3,915,647, 3,917,453, 3,963,357, 4,248,829, 4,255,384, 4,270,920 and 4,312,834, and U.K. Patent 2,052,057.

Preferably, the absorbent carrier material of the dry analytical element of this invention is a porous spreading zone. This zone can be self-supporting (i.e. composed of a material rigid enough to maintain its integrity), but preferably it is carried on a separate support. Such a support can be any suitable dimensionally stable, and preferably nonporous and transparent (i.e. radiation transmissive) material which transmits electromagnetic radiation of a wavelength between 200 and 900 nm. A support of choice for a particular element should be compatible with the intended mode of detection (fluorescence or colorimetric spectroscopy). Useful supports can be prepared from paper, metal foils, polystyrene, polyesters [e.g. poly(ethylene terephthalate)], polycarbonates and cellulose esters (.e.g. cellulose acetate).

The porous spreading zone can be prepared from any suitable fibrous or non-fibrous material or mixtures of either or both. The void volume and average pore size of this zone can be varied depending upon the use intended. Useful spreading zones can be prepared using either fibrous or nonfibrous materials, either mixed with a suitable binder material or woven into a fabric, as described in U.S. Patents 4,292,272, 3,992,158, 4,258,001 and 4,430,436 and Japanese Patent Publication 57(1982)-101760. It is desirable that the spreading zone be isotropically porous, meaning that the porosity is the same in each direction in the zone as caused by interconnected spaces or pores between particles, fibers or polymeric strands.

The elements can have two or more discrete zones, either in the sme layer or superimposed. At least one of which is preferably a porous spreading zone. The other zones can be reagent zones or registration zones as those zones are known in the art, additional spreading zones, radiation-blocking or filter zones, subbing zones or barrier zones. The zones are generally in fluid contact with each other, meaning that fluids, reagents and reaction products (e.g. color dyes) can pass or be transported between superposed regions of adjacent zones. In other words, when the element is contacted with fluid, all reagents of the analytical composition of this invention become mixed and can readily move within the element as a composition. Preferably, each zone is a separately coated layer, although two or more zones can be a single layer of the element.

A preferred embodiment of this invention is a multilayer element comprising a support having thereon, in order and in fluid contact, a first (e.g. registration) layer containing an indicator composition described herein (e.g. a colorimetric indicator composition) a second (e.g. reagent) layer containing creatine phosphate, AMP, ADP and a polyphosphate described herein, optionally a subbing layer, and a porous spreading layer which optionally contains either a CK activator or at least one antibody for the M subunits or both. The registration and reagent layers generally contain one or more hydrophilic binder materials (e.g. gelatin, vinyl pyrrolidone polymers and acrylamide polymers), surfactants and other addenda. The subbing layer can comprise one or more subbing materials known to one skilled in the art, e.g. vinyl pyrrolidone polymers or acrylamide polymers.

When the preferred colorimetric indicator composition described above is used, the registration layer also contains α-glycerophosphate oxidase, and the reagent layer also contains glycerol and glycerol kinase.

The elements of this invention can also contain one or more other addenda commonly put in the elements for various manufacturing or operational advantages. Such addenda include surfactants, buffers, solvents and hardeners.

In the elements of this invention, the coating coverage of creatine phosphate is at least 0.5 g/m$^2$, and preferably from 1 to 2 g/m$^2$. The amounts of the various components of the indicator composition are readily determined by one skilled in the art. The coating coverages for the polyphosphate, AMP and ADP are as follows, corresponding to the millimolar amounts described above:

polyphosphate (e.g. DAPP): at least 0.03 g/m$^2$, and preferably at least 0.05 g/m$^2$,

ADP: from 0.05 to 0.16 g/m$^2$, and preferably from 0.05 to 0.1 g/m$^2$.

AMP: at least a 10:1 molar ratio with ADP, and preferably at least 12:1 molar ratio and more preferably, at least a 14:1 molar ratio.

A variety of different elements, depending on the method of assay, can be prepared in accordance with the present invention. Elements can be configured in a variety of forms, including elongated tapes of any desired width, sheets, slides or chips.

The assay of this invention can be manual or automated. In general, in using the dry elements, CK-MB determination is made by taking the element from a supply roll, chip packet or other source and physically contacting it with sample (e.g. 1—200 μl) of the liquid to be tested. Such contact can be accomplished in any suitable manner, e.g. dipping or immersing the element into the sample or, preferably, by spotting the element by hand or machine with a drop of the sample with a suitable dispensing means.

After sample application, the element is exposed to any conditioning, such as incubation or heating, that may be desirable to quicken or otherwise facilitate obtaining any test result. Prior to incubation, a CK-activator and the antibodies for the M subunits can also be added to the element individually or together if they are not incorporated within the element.

The CK-MB added to the element in the test sample then catalyzes reaction of the ADP with the creatine phosphate substrate at a rate based on the amount of CK-MB present in the sample. The rate of detectable change (e.g. dye formation) due to either reaction of creatine phosphate or formation of the reaction product (e.g. ATP) is quantifiable by passing the elmeent through a zone in which suitable detection apparatus for reflection or transmission spectrophotometry, potentiometry or photometry, is provided. Suitable detection apparatus and procedures are known in the art.

The following examples illustrate the practice of this invention. As used in the context of this disclosure and the claims, I.U. represents the International Unit for enzyme activity defined as one I.U. being the amount of enzyme activity required to catalyze the conversion of 1 micromole of substrate per minute under standard pH and temperature conditions for the enzyme.

Examples 1 & 2—Assays of CK-MB using an analytical element

In this example, CK-MB was determined using dry analytical elements. Assays using Control elements outside of the scope of this invention were compared to assays using elements of this invention. All elements evaluated had the following format and components.

| | | |
|---|---|---|
| Spreading layer | Goat anti-human CK-MM | 22,900 U/m²** |
| | TiO₂ | 50 g/m² |
| | Cellulose acetate | 7 g/m² |
| | ESTANE polyurethane resin | 2.5 g/m² |
| | N-acetyl-L-cysteine | 0.4 g/m² |
| | TRITON X-405 surfactant | 1.7 g/m² |
| | Ethylenebis(oxyethylene-nitrilo)tetraacetic acid | 0.65 g/m² |
| Subbing layer | Poly(N-isopropylacrylamide) | 0.4 g/m² |
| Reagent layer | Gelatin (Hardened) | 5.4 g/m² |
| | Magnesium acetate | 0.65 g/m² |
| | TRITON X-200E surfactant | 0.1 g/m² |
| | Adenosine-5'-diphosphate (ADP) | * |
| | Glycerol kinase | 4320 I.U./m² |
| | Adenosine-5'-monophosphate (AMP) | * |
| | Creatine phosphate | 1.6 g/m² |
| | P¹,P⁵-di(adenosine-5')-pentaphosphate (DAPP) | * |
| | Glycerol | 0.2 g/m² |
| | 2-[Bis(2-hydroxymethyl)imino]-2-(hydroxymethyl)-1,3-propanediol | 2 g/m² |
| Registration layer | Gelatin (Hardened) | 5.4 g/m² |
| | 2-[Bis(2-hydroxyethyl)-imino]-2-(hydroxymethyl)-1,3-propanediol | 2 g/m² |
| | ALKANOL XC surfactant | 0.3 g/m² |
| | Peroxidase | 32,400 I.U./m² |
| | 2-(3,5-dimethoxy-4-hydroxyphenyl)-4,5-bis(4-dimethyl-aminophenyl)imidazole | 0.2 g/m² |
| | Ascorbic acid oxidase | 10,800 I.U./m² |
| | L-α-Glycerophosphate oxidase | 3240 I.U./m² |
| | Glycolic acid | 0.3 g/m² |
| | 5,5-Dimethyl-1,3-cyclohexane-dione | 0.05 g/m² |
| | TRITON X-200E surfactant | 0.1 g/m² |
| | 2,4-Di-n-pentylphenol | 2 g/m² |
| | Poly(ethylene terephthalate) Support | |

*See Table I below for amounts.

**The antisera level is given in Units (U) which are defined by the titer assay: (50% inhibition titer) (ml/0.093 m²)=U/m².

EP 0 200 541 B1

TABLE I

| Element | AMP (g/m²) | ADP (g/m²) | AMP:ADP molar ratio | DAPP (g/m²) |
|---|---|---|---|---|
| Control A*** | 1.08 | 0.08 | 14.7:1 | 0.054 |
| Control B | 0.22 | 0.16 | 1.5:1 | 0.001 |
| Control C | 0.22 | 0.16 | 1.5:1 | 0.004 |
| Control D | 1.08 | 0.08 | 14.7:1 | 0.004 |
| Control E | 1.08 | 0.08 | 14.7:1 | 0.010 |
| Example 1 | 1.08 | 0.08 | 14.7:1 | 0.032 |
| Example 2 | 1.08 | 0.08 | 14.7:1 | 0.054 |

***This element also contained 0.025 g/m² sodium fluoride which is not desirable in the practice of this invention.

Determination of CK-MB was made using each element according to the following procedure:

Test fluids containing 11.0—15.3 I.U./l human CK-MB activity in a heat-treated human serum pool base were used. Adenylate kinase isoenzymes were added to individual pools as follows: 110 I.U./l liver AK, 190 I.U./l erythocyte AK and 190 I.U./l muscle AK (final concentrations). Control fluids were serum pool base without AK.

Aliquots (10 μl) of each test fluid and control fluid were spotted on samples of the element illustrated above. Reflection densities produced from dye generated by the presence of CK-MB were monitored on a spectrophotometer over a period of 7 to 9 minutes at 37°C and 670 nm. CK-MB activities were predicted using a predetermined calibration curve.

The net bias resulting from the presence of each AK isoenzyme was calculated by obtaining the difference in predicted CK-MB activity of the AK-containing samples and a control using 5 replicates. For example, the predicted CK-MB activity in the sample containing liver AK was 10.68 I.U./l, while the predicted activity in the Control was 10.04 I.U./l. Therefore, the net bias was 0.64 I.U./l.

The net bias results of the determinations are provided in Table II below. The acceptable net bias is 1% of original AK activity. In this example, the acceptable net bias for each isoenzyme was: 1.1% for liver AK, 1.9% for each of muscle and erythrocyte AK.

TABEL II

| Element | Net bias (I.U./l) | | |
|---|---|---|---|
| | Liver AK | Erythrocyte AK | Muscle AK |
| Control A | 1.70 | 0.71 | 0.25 |
| Control B | 27.8 | 4.43 | 6.69 |
| Control C | 23.8 | 1.84 | 2.08 |
| Control D | 1.63 | 1.40 | 0.52 |
| Control E | 1.80 | 1.69 | 0.73 |
| Example 1 | 0.64 | 1.43 | 0.11 |
| Example 2 | 0.75 | 1.01 | 0.44 |

It is evident from the data that the assays using the analytical elements of this invention had acceptably low net biases (i.e. very low AK interference). That is, less than 1% of the original amount of each adenylate kinase isoenzyme, and especially liver AK remained effective in the assay. Control A, which contained sodium fluoride as an AK inhibitor, exhibited low interference from erythocyte and muscle isoenzymes, but the interference from liver AK was unacceptably high. Controls B—E, which contained insufficient amounts of AMP or DAPP, also exhibited unacceptably high AK interference from one or more of the isoenzymes.

10

**Claims**

1. An analytical composition for the determination of creatine kinase-MB while substantially all activity of isoenzymes of adenylate kinase is inhibited, the analytical composition comprising creatine phosphate and an indicator composition which is capable of providing a detectable change in response to the reaction of creatine phosphate or its reaction product,

the composition characterized as comprising, in the absence of fluoride ion, adenosine-5'-diphosphate in an amount of from 1 to 3 millimolar, and

a combination of adenylate kinase inhibitors consisting essentially of:

a) $P^1$, $P^5$-di(adenosine-5')polyphosphate present in an amount of at least 0.3 millimolar, and

b) adenosine-5'-monophosphate present in a molar ratio to adenosine-5'-diphosphate of at least 10:1.

2. A dry analytical element for the determination of creatine kinase-MB comprising an absorbent carrier material, and containing an analytical composition while substantially all activity of isoenzymes of adenylate kinase is inhibited, the analytical composition comprising creatine phosphate, and an indicator composition which is capable of providing a detectable change in response to the reaction of creatine phosphate or its reaction product.

the element characterized by comprising, in the absence of fluoride ion adenosine-5'-diphosphate in an amount of from 1 to 3 millimolar, and

a combination of adenylate kinase inhibitors consisting essentially of:

a) $P^1$,$P^5$-di(adenosine-5')polyphosphate present in an amount of at peast 0.3 millimolar, and

b) adenosine-5'-monophosphate present in a molar ratio to adenosine-5'-diphosphate of at least 10:1.

3. The element as claimed in claim 2 wherein the absorbent carrier material is a porous spreading zone carried on a support.

4. The element as claimed in claim 3 wherein the spreading zone contains antibodies for creatine kinase-MM.

5. A method for the determination of creatine kinase-MB in an aqueous liquid comprising the steps of:

(A) in the presence of at least one antibody for creatine kinase-MM, containing a sample of a liquid suspected of containing creatine kinase-MB with an analytical composition while substantially all activity of isoenzymes of adenylate kinase is inhibited, the analytical composition comprising, in the absence of fluoride ion,

creatine phosphate,

an indicator composition which provides a detectable change in response to the reaction of creatine phosphate or its reaction product,

adenosine-5'-diphosphate present in an amount of from 1 to 3 millimolar, and

a combination of adenylate kinase inhibitors consisting essentially of:

a) $P^1$,$P^5$-di(adenosine-5')polyphosphate present in an amount of at least 0.3 millimolar, and

b) adenosine-5'-monophosphate present in a molar ratio to adenosine-5'-diphosphate of at least 10:1, and

(B) determining the detectable change resulting from the presence of creatine-MB.

6. The method as claimed in claim 5 wherein the detectable change is a colorimetric change.

7. The invention as claimed in any of claims 1 to 6 wherein the $P^1$,$P^5$-di(adenosine-5')polyphosphate is $P^1$,$P^5$-di(adenosine-5')pentaphosphate present in an amount of at least 0.5 millimolar.

8. The invention as claimed in any of claims 1 to 7 wherein the indicator composition provides an optical density change in response to adenosine-5'-triphosphate or its reaction product.

9. The invention as claimed in any of claims 1 to 7 wherein the indicator composition is a colorimetric indicator composition comprising a substance having peroxidative activity and a leuco dye.

10. The invention as claimed in any of claims 1 to 9 wherein the adenosine-5'-diphosphate is present in an amount of from 1 to 2 millimolar.

11. The invention as claimed in any of claims 1 to 10 whereinuthe molar ratio of adenosine-5'-monophosphate to adenosine-5'-diphosphate is at least 14:1.

**Patentansprüche**

1. Analytische Zusammensetzung zur Bestimmung von Kreatinkinase-MB, bei praktische vollständiger Inhibierung der Aktivität von Isoenzymen von Adenylat-Kinase, enthaltend Kreatinphosphat und eine Indikatorzusammensetzung, die eine feststellbare Veränderung als Folge der Reaktion von Kreatinphosphat oder seinem Reaktionsprodukt zu liefern vermag, dadurch gekennzeichnet, daß sie in Abwesenheit des Fluoridions enthält: Adenosin-5'-diphosphat in einer 1 bis 3 millimolaren Menge sowie eine Kombination von Adenylat-Kinase-Inhibitoren, die im wesentlichen bestehen aus:

a) $P^1$,$P^5$-Di(adenosin-5')polyphosphat in einer mindestens 0,3 millimolaren Menge und

b) Adenosin-5'-monophosphat, das in einem molaren Verhältnis zu Adenosin-5'-diphosphat von mindestens 10:1 vorliegt.

2. Trockenes analytisches Element für die Bestimmung von Kreatinkinase-MB mit einem absorbierenden Trägermaterial und einem Gehalt an einer analytischen Zusammensetzung bei praktisch vollständiger Inhibierung der Aktivität von Isoenzymen der Adenylat-Kinase, wobei die analytische Zusammensetzung enthält Kreatinphosphat und eine Indikatorzusammensetzung, die eine feststellbare Veränderung als Folge der Reaktion von Kreatinphosphat oder seinem Reaktionsprodukt zu liefern vermag, dadurch gekennzeichnet, daß das Element in Abwesenheit des Fluoridions enthält Adenosin-5'-diphosphat in einer 1 bis 3 millimolaren Menge sowie eine Kombination von Adenylat-Kinase-Inhibitoren, die im wesentlichen bestehen aus:

a) $P^1,P^5$-Di(adenosin-5')polyphosphat in einer mindestens 0,3 millimolaren Menge und

b) Adenosin-5'-monophosphat, das in einem molaren Verhältnis zu Adenosin-5'-diphosphat von mindestens 10:1 vorliegt.

3. Element nach Anspruch 2, in dem das absorbierende Trägermaterial eine poröse Ausbreitzone auf einem Träger ist.

4. Element nach Anspruch 3, in dem die Ausbreitzone Antikörper für Kreatinkinase-MM enthält.

5. Verfahren zur Bestimmung von Kreatinkinase-MB in eine wäßrigen Flüssigkeit mit den Stufen:

(A) Inkontaktbringen einer Probe einer Flüssigkeit, von der vermutet wird, daß sie Kreatinkinase-MB enthält, in Gegenwart von mindestens einem Antikörper für Kreatinkinase-MM, mit einer analytischen Zusammensetzung bei praktisch vollständiger Inhibierung der Aktivität von Isoenzymen von Adenylat-Kinase, wobei die analytische Zusammensetzung in Abwesenheit des Fluoridions enthält:

Kreatinphosphat und eine Indikatorzusammensetzung, die als Folge auf eine Reaktion des Kreatinphosphates oder seines Reaktionsproduktes eine feststellbare Änderung herbeiführt,

Adenosin-5'-diphosphat in einer 1 bis 3 millimolaren Menge sowie

eine Kombination von Adenylat-Inhibitoren, die im wesenltichen bestehen aus:

a) $P^1,P^5$-Di(adenosin-5')polyphosphat in einer mindestens 0,3 millimolaren Menge und

b) Adenosin-5'-monophosphat, das in einem molaren Verhältnis zu Adenosin-5'-diphosphat von mindestens 10:1 vorliegt und

(B) Bestimmung der feststellbaren Veränderung, die sich aus dem Vorhandensein von Kreatin-MB ergibt.

6. Verfahren nach Anspruch 5, in dem die feststellbare Veränderung eine colorimetrische Veränderung ist.

7. Die Erfindung nach einem der Ansprüche 1 bis 6, in der das $P^1,P^5$-Di(adenosin-5')polyphosphat das $P^1,P^5$-Di(adenosin-5')pentaphosphat ist, das in einer millimolaren Menge von mindestens 0,5 vorliegt.

8. Die Erfindung nach einem der Ansprüche 1 bis 7, in der die Indikatorzusammensetzung als Folge auf Adenosin-5'-triphosphat oder seines Reaktionsproduktes eine Änderung der optischen Dichte herbeiführt.

9. Die Erfindung nach einem der Ansprüche 1 bis 7, in der die Indikatorzusammensetzung eine colorimetrische Indikatorzusammensetzung mit einer Substanz mit peroxidativer Aktivität und einem Leuco-Farbstoff ist.

10. Die Erfindung nach einem der Ansprüche 1 bis 9, in der das Adenosin-5'-diphosphat in einer 1 bis 2 millimolaren Menge vorliegt.

11. Die Erfindung nach einem der Ansprüche 1 bis 10, in der das molare Verhältnis von Adenosin-5'-monophosphat zu Adenosin-5'-diphosphat bei mindestens 15:1 liegt.

**Revendications**

1. Composition analytique pour la détermination de la créatine kinase-MB avec inhinition de pratiquement toute l'activité des iso enzymes de l'adénylate kinase, cette composition comprenant du phosphate de créatine et un indicateur capable de fournir un changement détectable en réponse à la réaction du phosphate de créatine ou à son produit de réaction, caractérisé en ce qu'elle comprend, en l'absence d'ions fluorure, de l'adénosine-5-diphosphate à une concentration de 1 à 3 millimolare, et une combinaison d'inhibiteurs de l'adénylate kinase consistant essentiellement en

a) $P^1,P^5$-di(adénosine-5')polyphosphate, à une concentration au moins 0,3 millimolaire et

b) adénosine-5'-monophosphate, dans un rapport molaire avec l'adénosine-5'-diphosphate au moins de 10:1.

2. Produit analytique sec pour la détermination de la créatine-kinase-MB comprenant un support absorbant, et contenant une composition analytique avec inhibition de pratiquement toute l'activité des isoenzymes de l'adénylate kinase, la composition analytique comprenant du phosphate de créatine et un indicateur capable de fournir un changement détectable en réponse à la réaction du phosphate de créatine ou à son produit de réaction, ce produit étant caractérisé en ce qu'il comprend, en l'absence d'ions fluorure, de l'adénosine-5-diphosphate à une concentration de 1 à 3 millimolaire, et une combinaison d'inhibiteurs de l'adénylate kinase consistant essentiellement en

a) $P^1,P^5$-di(adénosine-5')polyphosphate, à une concentration au moins 0,3 millimolaire et

b) adénosine-5'-monophosphate, dans un rapport molaire avec l'adénosine-5'-diphosphate au moins de 10:1.

3. Produit selon la revendication 2, dans lequel le support absorbant est une zone d'étalement poreuse sur un support.

4. Produit selon la revendication 2, dans lequel la zone d'étalement contient des anticorps de la créatine kinase-MM.

# EP 0 200 541 B1

5. Procédé pour la détermination de la créatine kinase-MB dans un liquide aqueux comprenant les étapes suivantes:

(A) on met en contact, en présence d'au moins un anticorps pour la créatine kinase-MM, un échantillon pouvant contenir de la créatine kinase-MB avec une composition analytique avec inhibition de pratiquement toute l'activité des isoenzymes de l'adénylate kinase, cette composition comprenant en l'absence d'ions fluorure,

du phosphate du créatine,

un indicateur capable de fournir un changement détectable en réponse à la réaction du phosphate de créatine ou à de son produit de réaction,

de l'adénosine-5-diphosphate à une concentration de 1 à 3 millimolaire, et

une combinaison d'inhibiteurs de l'adénylate kinase consistant essentiellement en

a) $P^1,P^5$-di(adénosine-5')polyphosphate, à une concentration d'au moins 0,3 millimolaire et

b) adénosine-5'-monophosphate, dans un rapport molaire avec l'adénosine-5'-diphosphate au moins de 10:1, et

(B) on détermine le changement détectable résultant de la présence de la créatine-MB.

6. Procédé selon la revendication 1, dans lequel le changement détectable est un chagement colorimétrique.

7. L'invention selon l'une des revendications 1 à 6, dans laquelle le $P^1,P^5$-di(adénosine-5')polyphosphate est le $P^1,P^5$-di(adénosine-5')pentaphosphate présent à une concentration au moins 0,5 millimolaire.

8. L'invention selon l'une des revendications 1 à 7, dans laquelle l'indicateur provoque un changement de densité optique en réponse à l'adénosine 5'-triphosphate ou à son produit de réaction.

9. L'invention selon l'une des revendications 1 à 7, dans laquelle l'indicateur est un indicateur colorimètrique comprenant une substance à activité péroxydante et un colorant leuco.

10. L'invention selon l'une des revendications 1 à 9, dans laquelle l'adénosine-5'-diphosphate est présent à une concentration de 1 à 2 millimolaire.

11. L'invention selon l'une des revendications 1 à 10, dans laquelle le rapport molaire de l'adénosine-5'-monophosphate à l'adénosine-5'-diphosphate est au moins de 14:1.